# EUROPÄISCHE PATENTANMELDUNG

(11) **EP 3 069 756 A1**
(43) Veröffentlichungstag der Anmeldung: **21.09.2016**
(21) Anmeldenummer: 16157062.7
(22) Anmeldetag: 24.02.2016
(51) Int. Cl.: A61N 1/375, A61N 1/39, A61N 1/362, A61N 1/05

(54) **DURCHFÜHRUNG EINES IMPLANTIERBAREN MEDIZINELEKTRONISCHEN GERÄTS, VERFAHREN ZUR HERSTELLUNG EINER SOLCHEN UND IMPLANTIERBARES MEDIZINELEKTRONISCHES GERÄT**

(30) Priorität: 20.03.2015 US 201562135711 P
(71) Anmelder: BIOTRONIK SE & Co. KG, 12359 Berlin (DE)
(72) Erfinder: Kronmüller, Daniel, 90409 Nürnberg (DE); Arnold, Michael, 91056 Erlangen (DE); Fries, Lilli, 90547 Stein (DE)
(74) Vertreter: Galander, Marcus

(57) **Zusammenfassung**

Durchführung eines implantierbaren medizinelektronischen Geräts, mit einem Keramikoder Glas-Isolierkörper, einem den Isolierkörper umfassenden Durchführungs-Flansch und mindestens einem den Isolierkörper durchstoßenden Anschlusselement zum externen Anschluss eines elektrischen oder elektronischen Bauelements des Geräts, wobei der Durch-führungs-Flansch aus mehreren vorgeformten Teilen gefiigt ist.

## Beschreibung

Die Erfindung betrifft eine Durchführung eines implantierbaren medizinelektronischen Gerätes sowie auch ein derartiges Gerät. Dieses umfasst typischerweise ein Gerätegehäuse, in dem elektronische und elektrische Funktionseinheiten untergebracht sind, einen Gerätekopf mit mindestens einer Elektrode oder einem Leitungsanschluss und eine zwischen Gerätegehäuse und Gerätekopf angeordnete Durchführung für mindestens ein die Elektroden oder den Leitungsanschluss mit einer Funktionseinheit verbindendes elektrisches Leiterelement. Eine derartige Durchführung umfasst einen Isolierkörper, insbesondere aus Keramik oder Glas, einen den Isolierkörper umfassenden Durchführungs-Flansch und mindestens ein den Isolierkörper durchstoßendendes Anschlusselement zum externen Anschluss eines elektrischen oder elektronischen Bauelements des Geräts. Des Weiteren betrifft die Erfindung ein Verfahren zur Herstellung einer solchen Durchführung.

Implantierbare Geräte der oben bezeichneten Art sind insbesondere als Herzschrittmacher oder implantierbare Kardioverter (speziell Defibrillatoren) seit langem in massenhaftem Einsatz. Es kann sich hierbei aber auch um eine weniger komplexe Vorrichtung, wie etwa eine Elektroden- oder Sensorleitung oder auch ein Cochlearimplantat, handeln.

Die meisten praktisch bedeutsamen implantierbaren medizinelektronischen Geräte sind dazu vorgesehen, über geeignet platzierte Elektroden elektrische Impulse an reizbares Körpergewebe abzugeben. Um diese Funktion auszuführen, sind im Gehäuse des Gerätes elektronische/elektrische Funktionseinheiten zur Erzeugung der Impulse und zur geeigneten Steuerung der Impulserzeugung untergebracht, und außen am Gerät sind unmittelbar Elektroden oder Anschlüsse für mindestens eine Elektrodenleitung vorgesehen, in deren distalem Endabschnitt die Elektroden zur Impulsübertragung an das Gewebe angebracht sind.

Die elektronischen/elektrischen Funktionseinheiten im Geräteinneren sind in einer Weise mit den äußeren Elektroden oder Elektrodenleitungsanschlüssen zu verbinden, die unter den speziellen Bedingungen des implantierten Zustandes eine absolut und dauerhaft verlässliche Funktion gewährleistet. Des Weiteren hat die Durchführung eines solchen Gerätes dessen dauerhafte Dichtigkeit unter diesen Bedingungen zu sichern.

Bekannt sind insbesondere Durchführungen, deren Grund- und Isolationskörper im Wesentlichen aus Keramik oder Glas besteht, wobei auch mehrschichtige bzw. mehrteilige Aufbauten unter Einsatz von Metallen oder Metalloxiden entwickelt wurden und eingesetzt werden. Derartige bekannte Durchführungen erfüllen weitgehend die an sie gestellten Anforderungen.

Gängige Praxis ist das Fräsen von Durchführungs-Flanschen aus Vollmaterial. Die Flansche werden auf einer mehrachsigen CNC-Maschine gefräst. Es können komplizierte Geometrien mit Hinterschnitten gefertigt werden. Bei der Herstellung kommen verschiedene Werkzeuge (Bohrer, Fräser, Nutenfräser, Radienfräser, usw.) seriell zum Einsatz.

Die Bearbeitungszeit wächst mit zunehmender Komplexität des Bauteiles an. Materialverbrauch und Maschinenzeit zur Fertigung eines Flansches sind sehr hoch. Je nach Bearbeitung entstehen kurze oder lange Späne. Da die Spanbildung die Rauheit des Werkstücks beeinflusst, müssen geeignete Maßnahmen (Hochdruckkühlmittel, Spezialfilter) getroffen werden um die Späne vom Werkstück abzuführen und die Oberfläche nicht zu beschädigen. Hohlräume im Körper sind nicht möglich.

Ein Durchführungs-Flansch kann alternativ auch mittels MIM-Verfahren (Metal Injection Moulding) hergestellt werden. Bei diesem Verfahren wird ein Gemisch aus Metallpulver und organischem Binder in eine Form gespritzt, ausgeformt und abschließend gesintert. Bei diesem Verfahren wird der Flansch als Ganzes in einem Schritt gefertigt. Die Herstellung von Hinterschneidungen oder Hohlräumen im Flansch ist prozessbedingt nicht möglich. Das Werkstück muss über Ausformungsschrägen verfügen, und häufig sind Spuren vom Auswerfer und Schließkante des Werkzeuges am Bauteil zu erkennen.

Das Ausstanzen und Tiefziehen von Durchführungs-Flanschen ist ebenfalls bekannt. Je nach physikalischen Eigenschaften des eingesetzten Metallblechs treten bei den erforderlichen Umformschritten z. T. erhebliche Rückstellkräfte auf, so dass üblicherweise mehrere Umformschritte bei unterschiedlich eingestellten Temperaturen vorzusehen sind. Dabei ist die Anzahl der erforderlichen rekristallisierenden Zwischenglühungen geringer. Auf eine gleichmäßige Erwärmung muss unbedingt geachtet werden; als Schmiermittel sind u.a. molybdän-sulfidhaltige Stoffe zu empfehlen. Um die optische Qualität des Bleches zu erhalten muss mit Einlegefolien aus Kunststoffen gearbeitet werden. Für die Fertigung von einfachen Geometrien (z.B. Schrittmachergehäusen) aus Titan hat sich das Verfahren als kostengünstigstes Verfahren etabliert.

Umgeformte Bleche haben hohe Genauigkeit aber nur geringe Bauteilkomplexität. Da das Ausgangsprodukt stets ein Blech ist, können komplexe Strukturen (z.B. Nuten, Hinterschneidungen) nur mit Aufwand hergestellt werden. Die Dichtheit ist bei einem integrierten Fertigungsprozess nur schwer zu gewährleisten, da das Halbzeugs mehrmals gefaltet oder stark umgeformt werden muss, so dass Leckpfade im Gefüge entstehen oder zwischen den einzelnen Blechebenen geöffnet bleiben.

Nach obigem liegt der Erfindung die Aufgabe zu Grunde, eine verbesserte Durchführung der eingangs spezifizierten Art anzugeben, die insbesondere bei relativ hoher Komplexität der Form auf vergleichsweise einfache und somit kostengünstige Weise in hoher Präzision gefertigt werden kann. Des Weiteren soll ein entsprechendes Herstellungsverfahren angegeben und ein relativ einfach und kostengünstig herzustellendes implantierbares medizinelektronisches Gerät bereitgestellt werden.

Diese Aufgabe wird in ihren Vorrichtungsaspekten durch eine Durchführung mit den Merkmalen des Anspruchs 1 bzw. durch ein implantierbares medizinelektronisches Gerät mit den Merkmalen des Anspruchs 15 gelöst. In ihren Verfahrensaspekten wird die Aufgabe durch Verfahren mit den Merkmalen der Ansprüche 8 - 10 gelöst. Zweckmäßige Fortbildungen sind Gegenstand der jeweiligen abhängigen Ansprüche.

Erfindungsgemäß ist bei der vorgeschlagenen Durchführung der Durchführungs-Flansch aus mehreren vorgeformten Teilen gefügt, wobei insbesondere mindestens zwei der vorgeformten Teile mittels eine stoffschlüssigen Verbindung, insbesondere einer Hartlötverbindung, oder einer Laserschweißverbindung gefügt sind bzw. mindestens eines der vorgeformten Teile ein vorgestanztes und gebogenes und/oder abgekantetes und/oder tiefgezogenes Blechteil ist.

In einer Ausführung der Erfindung ist das Blechteil insbesondere aus einem Titanblech, oder Titan-Legierungs-Blech gefertigt. Noch spezieller kann der Durchführungs-Flansch mehrere vorgeformte Blechteile unterschiedlicher Materialgüte umfassen, insbesondere aus Titan- oder Titan-Legierungs-Blech einerseits mit Grades 3 - 4 und andererseits mit Grades 1 - 2.

In weiteren Ausführungen umfasst gemäß einem ersten Aspekt der Durchführungs-Flansch einen mehrlagigen Blechteil-Verbund. Hierbei kann insbesondere in den mehrlagigen Blechteil-Verbund mindestens ein vorgeformtes Blechteil mit federelastischen Eigenschaften eingebunden sein, welches insbesondere in direkter Berührung mit dem Isolierkörper steht.

In weiteren Ausgestaltungen der Erfindung ist die Oberfläche des oder mindestens eines vorgeformten Blechteils in dessen Fügebereich strukturiert, insbesondere rillen- oder waffelförmig geprägt.

Das vorgeschlagene Verfahren umfasst einen Schritt des Laserschweißens zweier vorgeformter Teile des Durchführungs-Flansches im Vakuum oder unter Schutzgas. In einer alternativen Ausführung, oder auch kombiniert mit dem vorbenannten Schritt des Laserschweißens, umfasst das Verfahren einen Schritt des Hartlötens zweier vorgeformter Teile des Durchführungs-Flansches mittels eines Gold- oder Gold-Legierungs-Lotes, insbesondere in einem zusammenhängenden Fügeschritt mit einem Einfügen des Isolationskörpers in den Durchführungs-Flansch.

Nach einem relativ selbstständigen Verfahrensaspekt der Erfindung wird mindestens ein vorgeformtes Blechteil als Masterblech erzeugt, und es werden weitere Blechteile jeweils bezüglich des Masterblechs positioniert und insbesondere an dieses gefügt.

In einer Ausgestaltung der erwähnten, relativ unabhängigen Verfahrensaspekte werden vor und/oder während der Montage der Durchführung und/oder deren Verbindung mit einem Gerätegehäuse Klemmverbindungen zwischen geeignet vorgeformten Teilen des Durchführungs-Flansches untereinander und/oder mit dem Isolierkörper und/oder mit dem Gerätegehäuse zur korrekten Positionierung derselben genutzt.

In einer weiteren Ausgestaltung des vorgeschlagenen Verfahrens wird nach dem Fügeschritt ein Nachbehandlungsschritt zur Rekonstruktion der Passivierungsschicht des vorgeformten Teils des Durchführungs-Flansches ausgeführt, insbesondere als nasschemisches Beizen.

In praktisch bedeutsamen Applikationen der Erfindung ist das vorgeschlagene Medizinelektronische Gerät ausgebildet als Herzschrittmacher oder implantierbarer Kardioverter oder als Cochlearimplantat. Auf diese gerätetechnischen Anwendungen ist die Erfindung aber nicht beschränkt, sondern sie ist grundsätzlich auch in sonstigen Geräten einsetzbar, die eine gattungsgemäße Durchführung umfassen.

Die Unterbaugruppe Flansch besteht aus verschiedenen Stanz-, Blech- und Biegeteilen. Diese werden separat in großer Stückzahl aus geeignetem Titanblech (z.B. Grade 1) gestanzt. Je nach Anwendung und Funktionselement ist es möglich Teile bzw. Bleche, die eine starke Federwirkung erfordern, aus Titan mit einer anderen Güte zu fertigen (z.B. Grade 3, 4).

Mit der Erfindung lassen sich, zumindest in bestimmten Ausführungen (wie weiter oben beispielhaft erläutert), insbesondere einer oder mehrere der folgenden Vorteile erreichen:
- Minimierung des Materialeinsatzes,
- Verkürzung der Maschinenzeit zur Herstellung der Durchführung,
- Insgesamt Verringerung der Herstellungskosten, auch durch die Möglichkeit der wirtschaftlichen Herstellung von Durchführungs-Teilen als Stanzteile in großer Stückzahl und Option einer Integration eines Umformschrittes in den Stanzschritt.
- Möglichkeit des Hinzufügens von Form-Details (z. B. eines Einschweißbördels oder eines Spritzschutzes für den Schritt des Einschweißens der Durchführung in das Gehäuse),
- Möglichkeit einer Integration federnder Elemente oder von Anschlägen, Auflageflächen oder Klemmflächen zur temporären oder zusätzlichen Fixierung des Isolationskörpers oder Implantat-Gehäuses.
- Modularisierung des Durchführungs-Designs, mit dem Effekt, gewisse Module in hohen Stückzahlen verwenden und zu entsprechend niedrigen Preisen beziehen zu können.
- Option eines eigenständigeren, von Lieferanten unabhängigeren Durchführungs-Designs, bei späterer Veröffentlichung von Details und Verringerung der Gefahr eines Know-How-Abflusses.

Vorteile und Zweckmäßigkeiten der Erfindung ergeben sich im Übrigen aus der Beschreibung von Ausführungsbeispielen anhand der Figuren. Von diesen zeigen:
- Fig. 1: eine schematische, teilweise geschnittene Darstellung eines implantierbaren medizinelektronischen Geräts,
- Fig. 2: eine schematische Darstellung (Schnittansicht) eines Durchführungs-Flansches herkömmlicher Bauart,
- Fig. 3: eine perspektivische Darstellung einer Durchführung gemäß einem weiteren Ausführungsbeispiel der Erfindung,
- Fig. 4A bis 4F: schematische Querschnittsdarstellungen zur Erläuterung von Ausführungsvarianten der vorliegenden Erfindung,

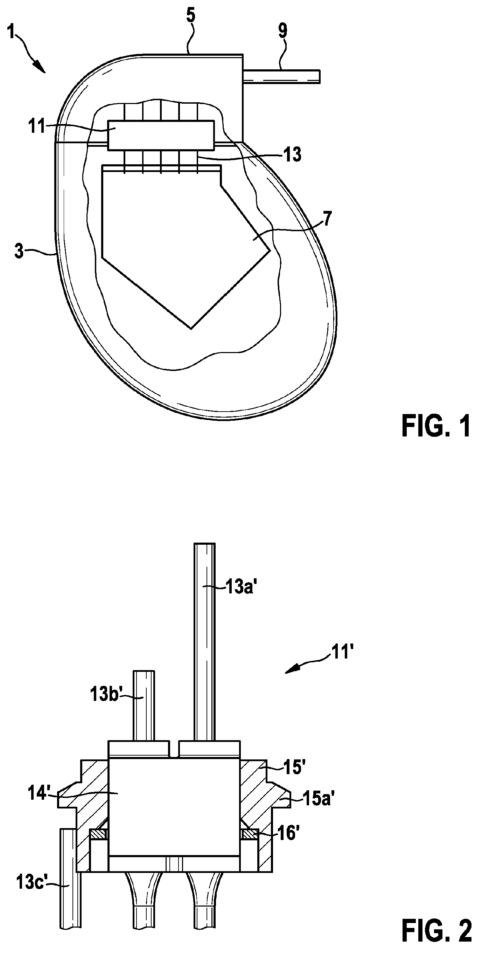

Fig. 1 zeigt einen Herzschrittmacher 1 mit einem Schrittmachergehäuse 3 und einem Kopfteil (Header) 5, in dessen Innerem neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11 umfasst eine Mehrzahl von Anschlussstiften 13. Die Anschlussstifte sind an einem Ende durch eine entsprechende Bohrung in der Leiterplatte gesteckt und mit dieser weich verlötet.

Fig. 2 zeigt in einer Schnittdarstellung längs einer mittigen Schnittebene eine Durchführung 11' mit herkömmlichem Aufbau, die einen Keramik-Isolationskörper 14' und einen aus Vollmaterial gedrehten Durchführungs-Flansch 15' umfasst, der den Isolationskörper umschließt. In einer den Isolationskörper ringförmig umgebenden Aussparung an der Unterseite des Durchführungs-Flansches 15' ist ein Lotring 16' eingelegt; dort wird der Isolationskörper mit dem Durchführungs-Flansch mittels eines Hartlötverfahrens hermetisch dicht verbunden. Lange und kurze Anschlussstifte 13a', 13b' durchstoßen den Isolationskörper 14', und ein Massepin 13c' ist draußen an den Durchführungs-Flansch 15' angeschweißt. Eine umlaufende Bördelkante 15a' am Durchführungs-Flansch 15' dient als Schweißkante, wenn der Flansch in eine Passung bzw. Bohrung eines (nicht dargestellten) Gerätegehäuses eingesetzt und dort verschweißt wird.

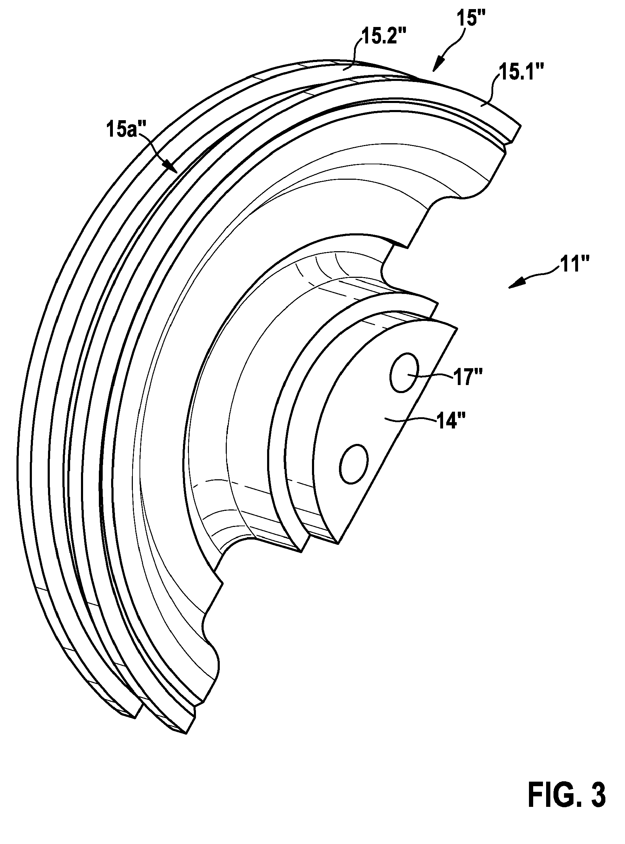

Fig. 3 zeigt, als perspektivische Ansicht, eine in der Draufsicht im Wesentlichen scheibenförmige Durchführung 11" eines medizinelektronischen Gerätes, die einen von einem Durchführungs-Flansch 15" umgriffenen Grund- und Isolierkörper 14" umfasst. Durchgangsöffnungen 17" im Grund- und Isolierkörper 14" sind zum Hindurchführen von (nicht gezeigten) Anschlussstiften vorgesehen. Der Durchführungs-Flansch 15" ist aus einem in eine komplexe Grundform geprägten oberen Blechteil 15.1 " und einem unteren Blechteil 15.2" zusammengesetzt, etwa durch Verschweißen oder Hartlöten. Die beiden Blechteile 15.1", 15.2" sind derartig geformt und zusammengefügt, dass sie am Außenumfang der Durchführung 15" zwischen sich einen umlaufenden Spalt 15a" bestimmen, in den eine innere Umfangskante eines (nicht gezeigten) Gerätegehäuses des medizinelektronischen Gerätes eingreifen kann und wo dieses Gehäuse mit der Durchführung verschweißt sein kann.

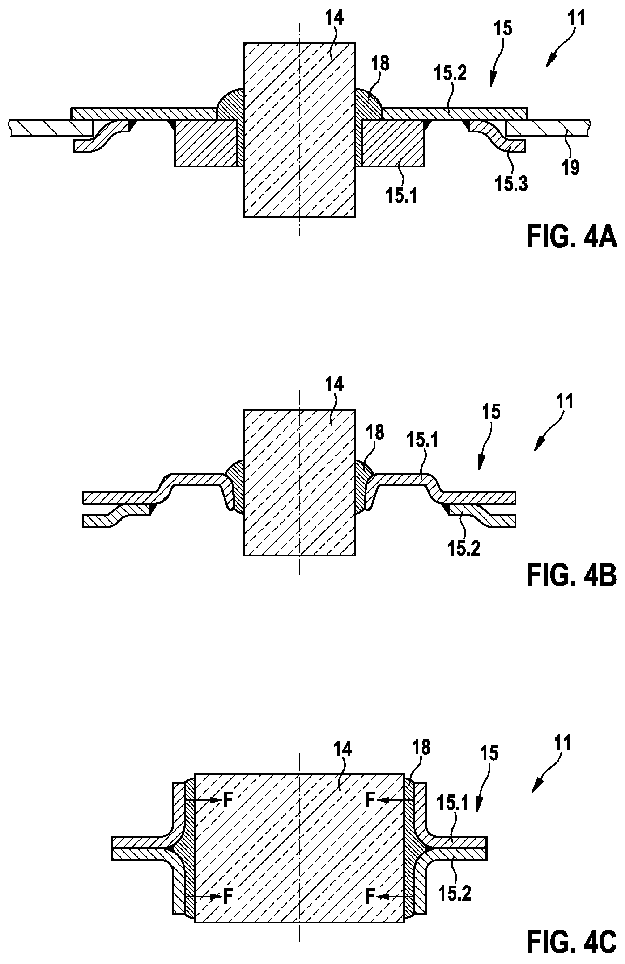

In den Figuren 4A - 4F sind verschiedene Ausführungsformen bzw. -aspekte der Erfindung skizzenartig in Art von Längsschnittdarstellungen verschiedener Durchführungen gezeigt. Obgleich die in diesen Figuren in Detailansichten skizzierten Durchführungen sich voneinander unterscheiden, sind sie in Anlehnung an Fig. 1 sämtlich mit Bezugsziffer 11 bezeichnet, und die Isolationskörper sind durchgängig mit Ziffer 14 und die Durchführungs-Flansche mit Ziffer 15 bezeichnet. Die Isolationskörper sind jeweils vereinfacht blockartig dargestellt; in der praktischen Ausführung werden hierin üblicherweise eines oder mehrere Anschlusselemente (Anschlusspins) eingebettet sein.

Gemäß Fig. 4A umfasst die Durchführung 11 neben dem Isolationskörper 14 einen Durchführungs-Flansch 15, der mittels einer Hartlötverbindung 18 mit dem Isolationskörper und mittels einer Laserschweißverbindung (nicht gezeigt) mit einem Gerätegehäuse 19 verbunden ist. Der Durchführungs-Flansch 15 ist hier aus drei Teilen gefügt, und zwar einem den Isolationskörper 15 ringförmig eng umgebenden ersten Flanschteil 15.1, einem auf dieses aufgeschweißten oder aufgelöteten zweiten Flanschteil (Blechteil) 15.2, und einem unter dessen Außenrand geschweißten dritten Flanschteil (gebogenen Blechteil) 15.3. Die Außenkanten des zweiten und dritten Flanschteils 15.2, 15.3 definieren einen Umfangsspalt, in den der Innenrand des Gerätegehäuses 19 eingreift.

Gemäß Fig. 4B umfasst die dort gezeigte Durchführung 11 neben dem Isolationskörper 14 einen zweiteiligen Durchführungs-Flansch 15 der aus einem ersten Blechteil 15.1 und einem zweiten Blechteil 15.2 gefügt ist. Das erste Blechteil 15.1 ist in seinem dem Isolationskörper benachbarten Randbereich ein erstes Mal und in einigem Abstand hiervon ein zweites Mal (entgegengerichtet) umgebogen, und das zweite Blechteil 15.2 ist außerhalb der zweiten Umbiegung von unten an das erste Blechteil angefügt. Durch die erste Umbiegung des ersten Blechteils 15.1 wird die Kontaktfläche mit der Lotverbindung 18 vergrößert, so dass diese leichter und mit höherer Zuverlässigkeit hergestellt werden kann.

Fig. 4C zeigt eine weitere Durchführung 11, bei der ein vergleichbarer Effekt erzielt wird, indem auch hier der Durchführungs-Flansch 15 mit einer vergrößerten Kontaktfläche zur Lotverbindung 18 versehen ist. Hier ist dies dadurch realisiert, dass der Flansch aus einem ersten und zweiten Blechteil 15.1, 15.2 gefügt ist, welche in ihrem inneren Randbereich in entgegengesetzte Richtungen abgekantet sind. Durch diese Abkantung wird zugleich eine elastische Anpresskraft F beider Blechteile in Richtung auf die Umfangsfläche des Isolationskörpers 14 realisiert.

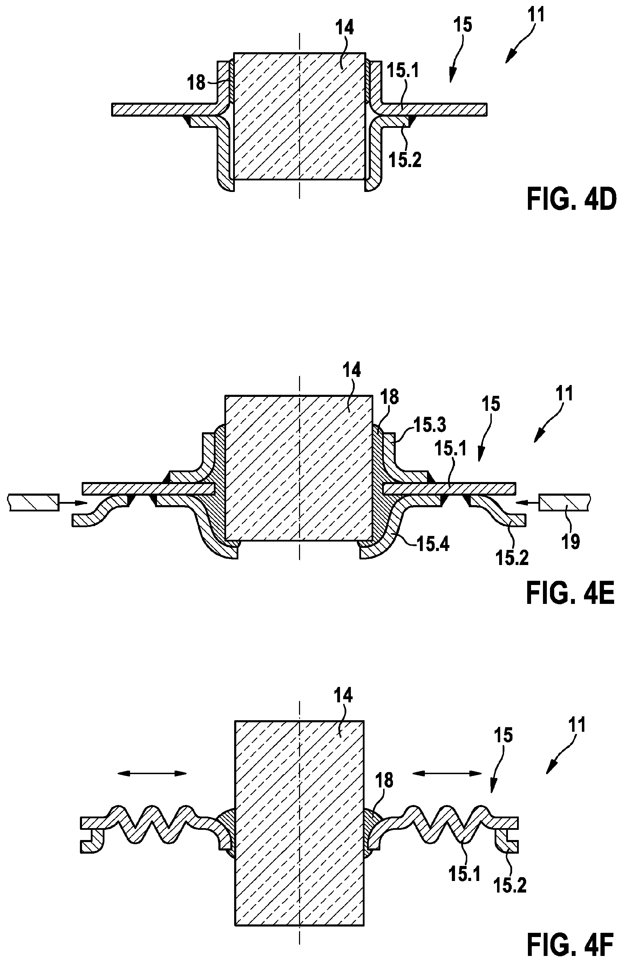

Fig. 4D zeigt eine weitere Ausgestaltung dieses Konstruktionsprinzips, wobei das zweite Blechteil 15.2 derart geformt ist, dass es mit seiner Innenkante den unteren Kantenbereich des Isolationskörpers 14 umgreift und dadurch eine zusätzliche Positionierungs- und Fixierungswirkung erbringt.

Fig. 4E zeigt eine Ausführung, die in gewisser Weise ähnlich der Ausführung nach Fig. 4 ist, und zwar insbesondere hinsichtlich der Bereitstellung eines Umfangsspaltes zwischen einem ersten, ebenen Blechteil 15.1 und einem mit diesem im äußeren Randbereich gefügten, nach unten gebogenen zweiten Blechteil 15.2. Außerdem ist, im Aufgreifen des in Fig. 4B bis 4D skizzierten und weiter oben beschriebenen Konzepts der Vergrößerung der Kontaktfläche des Flansches 15 mit der Lotverbindung 18, am Innenrand des ersten Blechteils 15.1 unten und oben jeweils ein weiteres Blechteil 15.3, 15.4 aufgesetzt. Ähnlich wie bei der Ausführung nach Fig. 4D, ist das unten aufgesetzte vierte Blechteil 15.4 so gestaltet, dass es mit einem umgebogenen Innenkantenbereich die untere Umfangskante des Isolationskörpers 14 umgreift.

In Fig. 4F ist eine Durchführung 11 gezeigt, deren Durchführungs-Flansch 15 aus zwei Blechteilen 15.1, 15.2 gefügt ist, wobei das erste Teil 15.1 zickzackförmig gebogen ist und hierdurch Elastizität in Pfeilrichtung, also senkrecht zur Umfangsfläche des Isolationskörpers 14, hat. Mit dieser Formgebung kann eine temporäre Fixierung des Isolationskörpers im Durchführungs-Flansch vor dem Erzeugen der Lotverbindung 18 realisiert werden.

Unter Verfahrensasnekten ist auf folgende Ausgestaltungen der Erfindung hinzuweisen: Bei Fertigung des Durchführungs-Flansches aus Blechteilen können diese zunächst in größerer Stückzahl aus Blech mit geeigneten Eigenschaften (z.B. Titanblech Grade 1) gestanzt werden. Anschließend oder im gleichen Produktionsschritt werden die Teile umgeformt. So können die notwendigen Geometrien (federnde Elemente, Nuten, Überlappstöße) in den Blechen integriert gefertigt werden.

Von Vorteil ist es, ein Masterblech herzustellen, welches zur Ausrichtung und Aufnahme der anderen Bleche dient. Die höchsten Toleranzen und kritischen Funktionen werden dabei idealerweise bereits im Masterblech realisiert. Anschließend werden die einzelnen Elemente bzw. Bleche automatisiert auf das Masterblech bestückt. Hierbei kann eine geeignete Vorrichtung oder ein Fertigungshilfsmittel zur Ausrichtung verwendet werden. Dies gewährleistet ein gleichbleibendes Toleranzfeld bzw. geringe Form- und Lagetoleranzen, bezogen auf das Masterblech.

Die Bleche werden miteinander oder mit dem Masterblech gefügt (z.B. punktgeschweißt). Um eine Versprödung oder Verunreinigung des Materials zu verhindern, muss Titan artgleich unter Ausschluss von Stickstoff, Sauerstoff und Wasserstoff verschweißt werden. Es ist daher notwendig, dass mit Schutzgas (z. B Argon min. 99,99%) oder unter Vakuum gefügt wird. Werden Funktionselemente am Masterblech angebracht, so ist häufig eine Punktschweißung oder Stoßnaht hinreichend. Das Lot zum Fügen des Keramikisolators kann mittels Klemmung zwischen mehreren Blechen im Flansch integriert werden.

Für das Handling in den anschließenden Prozessen können Hilfsbleche mit Trennkanten oder Sollbruchstellen angefügt werden. Die Sollbruchstellen sind konstruktiv so bemessen, dass sie beim fehlerhaften automatisierten Handling zerstört werden und so die automatisierte Montage von vorgeschädigten Teilen oder die Zerstörung von Bauteilen während des Einsetzens verhindern.

Durch eine integrierte optische Inspektion oder eine Schweißstromüberwachung können Fügedefekte erkannt und aussortiert werden.

Während des Fügens wird die natürliche Passivierungsschicht zerstört. Zunder, Anlauffarben, Ablagerungen von Metalloxiden und Schlacke können eine natürliche Selbstpassivierung verhindern, und so Keimstelle für eine spätere Korrosion sein. Um den Flansch nach dem Fügen erneut mit einer schützenden Passierungsschicht zu versehen, ist es sinnvoll das Bauteil zu beizen. Bekannte Beizlösungen enthalten typischerweise bis zu 4% Fluss- bzw. 25% Salpetersäure.

Das Fügen der Flanschbauteile kann teilweise auch im Anschlussprozess während des Hochtemperaturlötens erfolgen. Hierzu werden mindestens zwei Flanschbestandteile gefertigt und montiert. Während des Hochtemperaturlötens werden sie durch Lot (z. B. Gold) gefügt. Beim Fügen der Flanschelemente mit Gold müssen diese zueinander ausgerichtet, positioniert und ggf. fixiert werden. Beim Fügen der Teile mittels Hartlot handelt es sich um einen integrierten Fertigungsschritt in der Prozesskette. Dies heißt es fallen keine zusätzlichen Prozessschritte an. Da das gleichzeitige Fügen von mehreren Komponenten in einem Prozess kompliziert ist und die Gefahr der Produktion von Ausschuss sich multipliziert, eignet sich dieses Verfahren bevorzugt für unkritische Komponenten (z.B. Einpassung, Schweißkanten).

Die Ausführung der Erfindung ist auch in einer Vielzahl von Abwandlungen der hier gezeigten Beispiele und weiter oben hervorgehobenen Aspekte der Erfindung möglich.

## Patentansprüche

1. Durchführung (11) eines implantierbaren medizinelektronischen Geräts (1), mit einem Keramik- oder Glas-Isolierkörper (14`, 14"), einem den Isolierkörper (14`, 14") umfassenden Durchführungs-Flansch (15, 15', 15") und mindestens einem den Isolierkörper (14`, 14") durchstoßenden Anschlusselement (13) zum externen Anschluss eines elektrischen oder elektronischen Bauelements (7) des Geräts,
wobei der Durchführungs-Flansch (15, 15', 15") aus mehreren vorgeformten Teilen gefügt ist.

2. Durchführung (11) nach Anspruch 1, wobei mindestens zwei der vorgeformten Teile mittels eine stoffschlüssigen Verbindung, insbesondere einer Hartlötverbindung oder einer Laserschweißverbindung, gefügt sind.

3. Durchführung (11) nach Anspruch 1 oder 2, wobei mindestens eines der vorgeformten Teile ein vorgestanztes und gebogenes und/oder abgekantetes und/oder tiefgezogenes Blechteil, insbesondere aus einem Titanblech oder Titan-Legierungs-Blech, ist.

4. Durchführung (11) nach Anspruch 3, wobei der Durchführungs-Flansch (15, 15', 15") mehrere vorgeformte Blechteile unterschiedlicher Materialgüte umfasst, insbesondere aus Titan- oder Titan-Legierungs-Blech einerseits mit Grades 3 - 4 und andererseits mit Grades 1 - 2.

5. Durchführung (11) nach Anspruch 3 oder 4, wobei der Durchführungs-Flansch (15, 15', 15") einen mehrlagigen Blechteil-Verbund umfasst.

6. Durchführung (11) nach Anspruch 5, wobei in den mehrlagigen Blechteil-Verbund mindestens ein vorgeformtes Blechteil mit federelastischen Eigenschaften eingebunden ist, welches insbesondere in direkter Berührung mit dem Isolierkörper (14`, 14") steht.

7. Durchführung (11) nach einem der Ansprüche 3 bis 6, wobei die Oberfläche des oder mindestens eines vorgeformten Blechteils in dessen Fügebereich strukturiert, insbesondere rillen- oder waffelförmig geprägt, ist.

8. Verfahren zur Herstellung einer Durchführung (11) nach einem der vorangehenden Ansprüche, umfassend einen Schritt des Laserschweißens zweier vorgeformter Teile des Durchführungs-Flansches (15, 15', 15") im Vakuum oder unter Schutzgas.

9. Verfähren zur Herstellung einer Durchführung (11) nach einem der Ansprüche 1 bis 7, umfassend einen Schritt des Hartlötens zweier vorgeformter Teile des Durchführungs-Flansches (15, 15', 15") mittels eines Gold- oder Gold-Legierungs-Lotes, insbesondere in einem zusammenhängenden Fügeschritt mit einem Einfügen des Isolationskörpers (14', 14") in den Durchführungs-Flansch (15, 15', 15").

10. Verfahren zur Herstellung einer Durchführung (11) nach einem der Ansprüche 1 bis 7, wobei mindestens ein vorgeformtes Blechteil als Masterblech erzeugt wird und weitere Blechteile jeweils bezüglich des Masterblechs positioniert und insbesondere an dieses gefügt werden.

11. Verfahren nach einem der Ansprüche 8 bis 10, wobei vor und/oder während der Montage der Durchführung (11) und/oder deren Verbindung mit einem Gerätegehäuse (3, 19) Klemmverbindungen zwischen geeignet vorgeformten Teilen des Durchführungs-Flansches (15, 15', 15") untereinander und/oder mit dem Isolierkörper (14`, 14") und/oder mit dem Gerätegehäuse (3, 19) zur korrekten Positionierung derselben genutzt werden.

12. Verfahren nach einem der Ansprüche 8 bis 11, wobei nach dem Fügeschritt ein Nachbehandlungsschritt zur Rekonstruktion der Passivierungsschicht des vorgeformten Teils des Durchführungs-Flansches (15, 15', 15") ausgeführt wird, insbesondere als nasschemisches Beizen.

13. Implantierbares medizinelektronisches Gerät mit einer Durchführung (11) nach einem der Ansprüche 1 bis 7.

14. Gerät nach Anspruch 13, ausgebildet als Herzschrittmacher oder implantierbarer Kardioverter.

15. Gerät nach Anspruch 13, ausgebildet als Cochlearimplantat.
